(19) Europäisches Patentamt
European Patent Office
Office européen des brevets

(11) **EP 3 076 333 A1**

(12) **EUROPÄISCHE PATENTANMELDUNG**

(43) Veröffentlichungstag:
**05.10.2016 Patentblatt 2016/40**

(51) Int Cl.:
*G06K 9/00* (2006.01)         *G01N 33/49* (2006.01)
*G01N 21/49* (2006.01)        *G06K 9/62* (2006.01)
*G01N 15/14* (2006.01)

(21) Anmeldenummer: **15162478.0**

(22) Anmeldetag: **02.04.2015**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA**

(71) Anmelder:
• **Siemens Aktiengesellschaft**
**80333 München (DE)**

• **Siemens Healthcare Diagnostics Products GmbH**
**35041 Marburg (DE)**

(72) Erfinder: **Die Erfindernennung liegt noch nicht vor**

(74) Vertreter: **Maier, Daniel Oliver**
**Siemens AG**
**Postfach 22 16 34**
**80506 München (DE)**

(54) **ANALYSEVERFAHREN ZUR KLASSIFIKATION EINER TESTDISPERSION HINSICHTLICH DES VORLIEGENS EINER SICHELZELLENANÄMIE**

(57) Zusammenfassend betrifft die Erfindung ein Analyseverfahren zur Klassifikation einer Erythrozyten enthaltenden Testdispersion hinsichtlich des Vorliegens einer Sichelzellenanämie mit den Schritten
- Ermittlung von Messwerten ($P_i$) zu optischen Merkmalen ($M_i$) der zu klassifizierenden, Erythrozyten enthaltenden Testdispersion (12),
- Berechnung eines Klassifikationsindex (Y) mittels einer bereitgestellten, insbesondere linearen, Diskriminanzfunktion unter Verwendung einer Anzahl (n) der ermittelten Messwerte ($P_i$) und
- Klassifikation der Testdispersion (12) hinsichtlich des Vorliegens der Sichelzellenanämie in Abhängigkeit vom berechneten Klassifikationsindex (Y).

FIG 4

EP 3 076 333 A1

**EP 3 076 333 A1**

**Beschreibung**

Die Erfindung betrifft ein Analyseverfahren zur Klassifikation einer Erythrozyten (rote Blutkörperchen) enthaltenden Testdispersion hinsichtlich des Vorliegens einer Sichelzellenanämie, ein Computerprogrammprodukt, welches einen Computer zur Durchführung eines derartigen Klassifikationsverfahrens in die Lage versetzt und ein optisches Analysesystem zum Nachweis des Vorliegens einer Sichelzellenanämie anhand einer Erythrozyten enthaltenden Testdispersion, insbesondere einer Blutprobe.

Insbesondere im medizinischen Bereich werden heute automatische und halbautomatische optische Analyseverfahren eingesetzt, um biologische Proben hinsichtlich ihrer Zusammensetzung, hinsichtlich der Art und Beschaffenheit ihrer Bestandteile oder hinsichtlich des Vorliegens charakteristischer Merkmale als Indizien für Erkrankungen zu charakterisieren. Als biologische Proben werden hierbei üblicherweise Dispersionen in Form von Körperflüssigkeiten oder verflüssigtem Probenmaterial verwendet, wobei die disperse Phase häufig Zellen oder Zell- bzw. Gewebematerial biologischen Ursprungs umfasst. Typische Proben sind beispielsweise Blutproben, Blutplasmaproben, Liquorproben, Speichelproben, Urinproben oder in Flüssigkeiten aufbereitetes Zellen- bzw. Gewebematerial. Das Dispersionsmedium als flüssige Phase kann durch die Körperflüssigkeit selbst gestellt sein. Die flüssige Phase kann jedoch auch ein hinzugefügtes Medium wie beispielsweise Wasser sein, oder ein Nährmedium, ein chemisches Lösungs-, Aufbereitungs- oder Indikationsmittel umfassen oder daraus bestehen.

Die optische Analyse einer biologischen Probe wird typischerweise in halbautomatischen oder automatischen Analysegeräten durchgeführt, wobei Licht spezifischer Wellenlängen und spezifischer Intensität, insbesondere Laserlicht, in die Dispersion fokussiert und durch die Dispersion verursachte Änderungen beobachtet werden. Beispielsweise kann eine Spektralanalyse vorgenommen werden. Das Licht kann in Transmissionsrichtung oder Reflexionsrichtung beobachtet werden. Auch können Streurichtungen beobachtet werden. Für charakteristische Wellenlängen oder über das vorhandene Spektrum gemittelt kann die Intensität des aus verschiedenen Richtungen beobachteten Lichts ermittelt werden. Die Messungen können orts- und zeitaufgelöst erfolgen. Ganz allgemein werden durch die optischen Analyseverfahren Messwerte zu bestimmten optischen Merkmalen der untersuchten Dispersionen ermittelt.

Aus der WO 00/58727 ist beispielsweise ein optisches Analyseverfahren für Blutproben bekannt, mit dem eine automatisierte Blutzellen-Analyse einschließlich des Feststellens der Anzahl von Leukozyten und insbesondere von Retikulozyten durchgeführt werden kann. Nach Hinzufügen von Reaktionsmitteln, Färbemitteln und Sphärungs- bzw. Rundungsmitteln für Erythrozyten werden die Streu- und Absorptionseigenschaften der derart behandelten Blutprobe untersucht. Aus den beobachteten Streu- und Absorptionsdaten werden die unterschiedlichen Blutzellen diskriminiert und analysiert.

Mittels moderner automatisierter optischer Analyseverfahren lassen sich anhand der Vielzahl der bestimmten optischen Merkmale auch sehr komplexe charakteristische Eigenschaften der untersuchten Proben feststellen. Insbesondere können festgestellte charakteristische Merkmale auch als Indizien für das Vorliegen einer Erkrankung der Person gewertet werden, der die untersuchte Probe entnommen wurde.

Dennoch sind zur Feststellung spezifischer und insbesondere hinsichtlich der Häufigkeit ihres Auftretens und der Sterblichkeitsrate relevanter Krankheiten wie beispielsweise der Malaria oder der Sichelzellenanämie nach wie vor manuelle Auswerte- und Zählverfahren notwendig, um eine verlässliche Diagnose treffen zu können. Sowohl bei der Malaria als auch bei der Sichelzellenanämie muss in Blutausstrichen mikroskopisch eine jeweils charakteristische Form der befallenen bzw. betroffenen Erythrozyten oder im Falle der Malaria gegebenenfalls die Erreger selbst erkannt und ausgezählt werden. Bei diesen labordiagnostischen Verfahren helfen zwar Farbstoffe, die Zelltypen oder Erreger spezifisch einfärben, oder Zusatzstoffe, die zu einer Zersetzung (Lyse) von Zellen führen, in der mikroskopischen Erkennung der charakteristischen Merkmale. Dennoch ist das manuelle Auszählen der unter dem Mikroskop erkennbaren Artefakte ein äußerst aufwändiges, zeitintensives und daher teures Analyseverfahren.

Der Erfindung liegt die Aufgabe zugrunde, ein optisches Analyseverfahren für eine Erythrozyten enthaltende Testdispersion bereit zu stellen, welches eine automatisierte Klassifikation hinsichtlich des Vorliegens der Sichelzellenanämie ermöglicht.

Diese Aufgabe wird erfindungsgemäß gelöst durch ein Analyseverfahren zur Klassifikation einer Erythrozyten enthaltenden Testdispersion, wobei Messwerte zu optischen Merkmalen der zu klassifizierenden, Erythrozyten enthaltenden Testdispersion ermittelt werden, wobei mittels einer bereitgestellten, insbesondere linearen, Diskriminanzfunktion unter Verwendung einer Anzahl der ermittelten Messwerte ein Klassifikationsindex berechnet wird, und wobei die Testdispersion hinsichtlich des Vorliegens der Sichelzellenanämie in Abhängigkeit vom berechneten Klassifikationsindex klassifiziert wird.

Bei der Testdispersion handelt es sich insbesondere um eine Dispersion mit einem flüssigen Dispersionsmedium. Im flüssigen Dispersionsmedium, das beispielsweise im Falle einer Blutprobe oder Liquorprobe durch die entnommene Körperflüssigkeit selbst zur Verfügung gestellt ist, sind die zum Klassifizieren hinsichtlich des Vorliegens einer Sichelzellenanämie erforderlichen Erythrozyten als disperse Phase enthalten. Das flüssige Dispersionsmedium sollte insbesondere in der Lage sein, die Testdispersion während der Ermittlung von Messwerten zu den optischen Merkmalen in einem flüssigen Zustand zu halten. Der Testdispersion können Zusätze zur Färbung, zur chemischen Veränderung, zur Zersetzung oder Auflösung von Zellen oder Zellbestandteilen oder sonstige Zusätze wie beispielsweise Gerinnungshemmer oder Verdünnungsmittel etc. beigegeben sein oder während der Durchführung des Analyseverfahrens beigegeben werden. Insofern sollte die Testdispersion für die Durchführung des Analyseverfahrens also verdünnbar, durchmischbar und/oder einschließlich der enthaltenen Erythrozyten auch transportierbar sein.

Die Sichelzellenanämie selbst tritt als Folge einer vererbbaren Genmutation phänomenologisch in einem veränderten Hämoglobin-Protein auf, welches insbesondere bei abnehmendem Sauerstoffpartialdruck zur Polymerisation neigt. Als Folge der Polymerisation der Hämoglobinmoleküle bilden sich charakteristische stäbchen- oder sichelzellenförmige Erythrozyten. Diese Erythrozyten verlieren ihre Elastizität und führen zu Verschlüssen in Kapillaren. Die Sauerstoffaufnahme ist reduziert.

An Sichelzellenanämie erkrankte Personen zeigen eine Resistenz gegenüber Malaria. In Gebieten mit Malaria ist die Mutation der Sichelzellenanämie sehr häufig, da sie den Trägern dort einen Evolutionsvorteil verschafft. Die Sichelzellenanämie tritt in heterozygoter und homozygoter Form auf. In der homozygoten Form sind die betroffenen Personen Träger zweier kranker, von den Eltern vererbter Allele. Sie stellen nur mutiertes krankhaft verändertes Hämoglobin her. Die betroffene Person leidet an gefährlichen Gefäßverschlüssen und einer grundsätzlich verringerten Sauerstoffaufnahme. Bei der heterozygoten Form sind die betroffenen Personen Träger eines gesunden und eines kranken Allels. Sie stellen sowohl gesundes als auch mutiertes Hämoglobin her. Die charakteristische Sichelzellenform tritt bei der heterozygoten Form vermehrt bei Absinken des Sauerstoffpartialdrucks im Blut auf, was insbesondere bei körperlicher Anstrengung oder in großen Höhen der Fall ist. Unter normalen Umständen ist lediglich 1 % der Erythrozyten betroffen.

Die Erfindung geht in einem ersten Schritt von der Überlegung aus, die durch automatisierte Analyseverfahren zur Verfügung gestellten Messwerte einer Vielzahl von optischen Merkmalen der untersuchten Probe durch Anwendung der an sich bekannten mathematischen Methode der Diskriminanzanalyse zur Klassifikation hinsichtlich des Vorliegens der Sichelzellenanämie heranzuziehen. Ausschlaggebend für diese Idee ist im Besonderen die durch eigene Untersuchungen gewonnene überraschende Erkenntnis, dass die üblicherweise in hämatologischen Analyseverfahren eingesetzten Zusätze zur Sphärisierung (Rundung) der Erythrozyten (die Streuparameter werden richtungsunabhängig) die durch Sichelzellenanämie verformten Erythrozyten nicht oder in nur geringem Maße in ihrer Form verändern. Folglich verbleibt die Information über verformte Erythrozyten als Folge der Sichelzellenanämie während der Durchführung eines automatisierten optischen Analyseverfahrens insbesondere in einer Blutprobe erhalten, so dass sich eine Testdispersion einer gesunden von der Testdispersion einer erkrankten Person in den Messwerten der optischen Merkmale prinzipiell unterscheiden muss.

In einem zweiten Schritt erkennt die Erfindung, dass der Einsatz der Diskriminanzanalyse eine insbesondere automatisierbare Möglichkeit bietet, durch Betrachtung einer spezifischen Kombination der Messwerte der optischen Merkmale ein Kriterium anzugeben, welches erlaubt, die Testdispersion hinsichtlich des Vorliegens der Sichelzellenanämie zu klassifizieren, sie also von einer Testdispersion einer gesunden Person zu unterscheiden. Während gegebenenfalls jedes einzelne optische Merkmal der Testdispersion nicht in der Lage ist, durch den jeweils festgestellten Messwert, der eine natürliche Streubreite um den Erwartungswert aufweist, ein Indiz für das Vorliegen der Sichelzellenanämie zu liefern, gelingt es durch Anwendung der Diskriminanzanalyse analytisch, in einer entsprechend gewichteten Kombination der ermittelten optischen Merkmale ein solches Unterscheidungskriterium hinsichtlich der Zuweisung der untersuchten Testdispersion zu einer an Sichelzellenanämie erkrankten oder gesunden Person zu finden. Insbesondere bietet die lineare Diskriminanzanalyse ein erstaunlich sicheres und analytisch brauchbares Werkzeug, wobei durch eine gewichtete Linearkombination der von optischen Merkmalen ermittelten Messwerte der Testdispersion ein Klassifikationsindex ermittelt werden kann, dessen Wert, wie für die Diskriminanzanalyse mathematisch bewiesen, eine Klassifikation der Testdispersion ermöglicht.

Die Diskriminanzanalyse ist an sich nicht Gegenstand der vorliegenden Anmeldung. Es soll jedoch hierzu nur angemerkt werden, dass es sich bei der Diskriminanzanalyse die zur Zuordnung eines Objekts (vorliegend der Testdispersion) zu zwei Gruppen (vorliegend die Gruppe der an Sichelzellenanämie Erkrankten und die Gruppe der Gesunden) eingesetzt wird, um eine analytische Methode handelt, die aus bekannten, der jeweiligen Gruppen zugeordneten Objekten in einem durch die betrachteten Merkmale aufgespannten mehrdimensionalen Raum eine Trennebene oder Trennlinie ermittelt, die die beiden Gruppen mit größter Wahrscheinlichkeit voneinander trennt. Ein Objekt jenseits der Trennebene kann der einen Gruppe und ein Objekt diesseits der Trennebene kann der anderen Gruppe zugeordnet werden. Anschaulich kann auch der Abstand eines untersuchten Objekts im mehrdimensionalen Raum bezüglich der jeweils aus den bekannten Objekten für eine Gruppe ermittelten Schwerpunkten bestimmt werden. Der jeweils kürzere Abstand bestimmt dann die Gruppenzugehörigkeit des untersuchten Objekts. Analytisch mathematisch kann auch ein Schwellwert angegeben werden, der in der vorgenannten Interpretation durch einen Abstand zum Schwerpunkt einer Gruppe definiert sein kann. Ist das untersuchte Objekt durch einen Wert (Abstand) größer oder kleiner des Schwellwerts charakterisiert, so kann dieses Objekt der einen oder der anderen Gruppe zugeordnet werden.

In der insbesondere linearen Diskriminanzanalyse wird aus Trainingsmesswerten von Objekten bekannter Zuordnung eine Diskriminanzfunktion als gewichtete Linearkombination aus den jeweiligen Messwerten gebildet. Der Gewichtungs- oder Signifikanzvektor bzw. die jeweiligen als Signifikanzparameter im Folgenden bezeichneten Einträge dieses Vektors werden mathematisch durch ein Optimierungsverfahren gelöst. Unter Verwendung der derart festgelegten optimierten Signifikanzparameter kann dann aus den Messwerten für das zu untersuchende Objekt konkret ein Klassifikationsindex bestimmt werden. Dieser Klassifikationsindex legt die Zuweisungen des untersuchten Objekts zu der einen oder der anderen Gruppe fest.

Da bekannte automatisierte optische Analyseverfahren für eine biologische Probe eine Vielzahl von optischen Merkmalen betrachten bzw. die hierzu entsprechenden Messwerte ermitteln, kann es vorteilhaft sein, die Auswahl der für die Diskriminanzanalyse betrachteten optischen Merkmale zu begrenzen. Dabei können beispielsweise bestimmte optische Merkmale ausgewählt werden, die eine gewisse Signifikanz für das Vorliegen der Sichelzellenanämie enthalten könnten. Dazu können technische Hintergründe oder technisches Vorwissen oder Erfahrungswerte herangezogen werden. Beispielsweise können im Fall der Sichelzellenanämie insbesondere diejenigen optischen Merkmale herangezogen werden, die in Verbindung mit Erythrozyten stehen bzw. typischerweise zur Diskriminierung der Erythrozyten gegenüber anderen Bestandteilen oder Zellarten verwendet werden. Jedoch können auch andere der bestimmten optischen Merkmale für die Diskriminanzanalyse herangezogen werden, deren Signifikanz sich durch Testreihen erst empirisch erschließen lässt. So kann die Anzahl der für die Diskriminanzanalyse verwendeten optischen Merkmale zum einen beschränkt und zum anderen die Qualität der Dikriminanzanalyse und insbesondere der vorzunehmenden Klassifikation hinsichtlich des Vorliegens der Sichelzellenanämie verbessert werden.

Vorteilhafterweise wird demnach der Klassifikationsindex aus einer Anzahl der Messwerte zu den jeweiligen optischen Merkmalen mittels durch die Diskriminanzfunktion für die optischen Merkmale bereitgestellten Signifikanzparametern ermittelt. Dabei ist die Anzahl der für die Berechnung des Klassifikationsindex herangezogenen optischen Merkmale zweckmäßigerweise gegenüber den von einem automatisierten optischen Analyseverfahren üblicherweise bereitgestellten Anzahl reduziert. Die Signifikanzparameter legen zum einen die Gewichte bzw. die Signifikanz für die einzelnen optischen Merkmale fest. Zum anderen stellen die bereitgestellten Signifikanzparameter aber auch die Lösung der oben genannten Optimierungsaufgabe der Diskriminanzanalyse dar, so dass die bereitgestellte Diskriminanzfunktion, die durch die ermittelten Signifikanzparameter wesentlich definiert ist, sogleich im Rahmen der gegebenen Messwerte die größtmögliche Unterscheidungssicherheit hinsichtlich der Klassifikation angibt.

Bevorzugt wird der Klassifikationsindex mittels durch die Diskriminanzfunktion für die optischen Merkmale bereitgestellten statistischen Größen, insbesondere Mittelwerte und Standardabweichungen, ermittelt. Die statistischen Größen beziehen sich hierbei auf einen Datensatz von Messwerten zu Dispersionen bekannter Klassifikation, den sogenannten Trainingsmesswerten. Insbesondere kann hierbei zwischen den statistischen Größen aus dem Datensatz von Messwerten zu den optischen Merkmalen von Dispersionen von an Sichelzellenanämie erkrankten Personen und von Dispersionen von gesunden Personen unterschieden werden. Es kann sich hierbei aber auch um die statistischen Größen des Datensatzes von Messwerten zu Dispersionen nur einer Gruppe handeln, so dass der Klassifikationsindex anschaulich gesehen den "Abstand" der untersuchten Testdispersion beispielsweise zu einem Zentrum, zu einem Erwartungswert oder zu einem Mittelwert von Messwerten zu den optischen Merkmalen dieser Dispersionen angibt. Als statistische Größen können neben Mittelwerten und Standardabweichungen insbesondere auch Varianzen oder Kovarianzen ermittelt und/oder bereitgestellt werden.

In einer bevorzugten Ausgestaltung wird der Klassifikationsindex berechnet durch:

$$Y = \sum_{i=1}^{n} l_i * (P_i - E_i)/\sigma_i,$$

wobei Y den Klassifikationsindex, n die Anzahl der optischen Merkmale der Testdispersion, $l_i$ die Signifikanzparameter der jeweiligen optischen Merkmale, $P_i$ die Messwerte der jeweiligen optischen Merkmale, $E_i$ die Mittelwerte der jeweiligen optischen Merkmale und $\sigma_i$ die Standardabweichung der jeweiligen optischen Merkmale bezeichnet.

In einer weiter bevorzugten Ausgestaltung der Erfindung sind oder wurden die optimierten Signifikanzparameter und die statistischen Größen der bereitgestellten Diskriminanzfunktion aus Trainingsmesswerten zu den optischen Merkmalen in Dispersionen bekannter Klassifikation ermittelt. Die statistischen Größen werden hierbei unmittelbar aus den Trainingsmesswerten zu den optischen Merkmalen der den unterschiedlichen Gruppen zugeordneten Dispersionen in an sich bekannter Art und Weise gebildet. Die Signifikanzparameter lassen sich mathematisch durch die der Diskriminanzanalyse zugrundeliegenden Optimierungsaufgabe ermitteln. Grundsätzlich ist dabei festzustellen, dass die Qualität der aus den Trainingsmesswerten ermittelten Diskriminanzfunktion hinsichtlich der Wahrscheinlichkeit einer richtigen Klassifikation um so besser wird, je größer der zugrundegelegte Datensatz der Trainingsmesswerte ist.

Vorliegend werden vorteilhafterweise die statistischen Größen der optischen Merkmale aus ersten Trainingsmesswerten ermittelt, wobei die ersten Trainingsmesswerte mit Dispersionen mit einer bezüglich des Vorliegens der Sichelzellenanämie negativen Klassifikation und die zweiten Trainingsmesswerte von Dispersionen mit einer bezüglich des Vorliegens der Sichelzellenanämie positiven Klassifikation abgeleitet sind. Bevorzugt sind die Signifikanzparameter aus den ersten und aus den zweiten Trainingsmesswerten mittels einer Diskriminanzanalyse abgeleitet, wobei insbesondere ein Bayes'sches Theorem verwendet wird.

Dabei wird der Klassifikationsindex insbesondere als eine Zufallsvariable interpretiert, wobei eine Wahrscheinlichkeit des Vorliegens einer Klassifikation über die Häufigkeit des Klassifikationsindex aufgetragen werden kann. Auf diese Weise liefert der Klassifikationsindex eine Wahrscheinlichkeitsverteilung, die sowohl Testdispersionen mit positiver als auch negativer Klassifikation beinhaltet, oder allgemein, mit allen vorgesehenen Klassifikationen. Zum Verwenden der Bayes'schen Theorie werden beispielsweise priore und posteriore Wahrscheinlichkeit in die Berechnung mit einbezogen. Dabei muss/müssen genügend Wissen beziehungsweise Trainingsmesswerte von verlässlichen priore Wahrscheinlichkeiten vorliegen, damit die Theorie zum Tragen kommt. Hierbei werden als priore Wahrscheinlichkeiten solche Wahrscheinlichkeiten angesehen, die mit Vorwissen geschätzt sind und posteriore Wahrscheinlichkeiten als Wahrscheinlichkeiten die nachträglich errechnet wurden.

Bei einer besonderen Ausführungsform kann eine Klassifikationsregel angegeben werden, aufgrund derer Wertebereiche des Klassifikationsindex einer bestimmten Klassifikation zugeordnet werden können. Insbesondere im Fall einer Zweiklassen-Klassifikation kann ein Schwellwert für den Klassifikationsindex angegeben werden, der den Wertebereich des Klassifikationsindex in zwei Intervalle aufteilt und somit zu einer eindeutigen Klassifikationsregel führt. Bei Unterschreitung des angegebenen Schwellenwerts erfolgt eine Klassifikation in die eine Klasse oder Gruppe; bei Überschreitung des Schwellwertes wird die Klassifikation in die andere Klasse oder Gruppe vorgenommen.

Bevorzugt wird ein Schwellwert $Y_S$ zur Klassifikation verwendet, wobei hinsichtlich des Vorliegens der Sichelzellenanämie ein Wert des Klassifikationsindex von $Y > Y_S$ einer positiven Klassifikation und von $Y < Y_S$ einer negativen Klassifikation zugeordnet ist. Insbesondere kann der Schwellwert durch Umformen der Diskriminanzfunktion auch so festgelegt werden, dass ein positiver Klassifikationsindex eine Zuordnung der Testdispersion zu einer an Sichelzellenanämie erkrankten Person, und ein negativer Klassifikationsindex die Zuordnung der Testdispersion zu einer gesunden Person anzeigt.

In einer zweckmäßigen Ausgestaltung des Analyseverfahrens werden zur Berechnung des Klassifikationsindex wenigstens zwei optische Merkmale berücksichtigt, wobei ein erstes optisches Merkmal ein Maß für eine optische Streuung in eine transversale X-Richtung zu einem Analysestrahl ist und ein zweites optisches Merkmal ein Maß für eine optische Streuung in einer zur X-Richtung senkrechten und zum Analysestrahl senkrechten Y-Richtung ist, wobei der Analysestrahl durch die Testdispersion verläuft. Der Analysestrahl verläuft hierbei im Wesentlichen entlang einer Z-Richtung, die sowohl auf der X-, als auch auf der Y-Richtung senkrecht steht. In der Z-Richtung kann der Analysestrahl beispielsweise durch optische Elemente wie Linsen, Filter oder dergleichen fokussiert, aufgeweitet oder anderweitig konditioniert werden, um in der Testdispersion eine gewünschte Strahleigenschaft aufzuweisen. Nach Durchgang durch die Testdispersion wird der Analysestrahl ausgewertet, wobei beispielsweise eine Nahfeldstreuung, eine Fernfeldstreuung und/oder Wellenlängenabsorptionen im Sinne einer Spektralanalyse berücksichtigt werden. Aus diesen Streuparametern kann insbesondere auf das Vorliegen der für die Sichelzellenanämie charakteristisch verformten Erythrozyten geschlossen werden bzw. weisen diese Streuwerte gegebenenfalls eine hohe Signifikanz hinsichtlich einer Klassifikation bezüglich des Vorliegens einer Sichelzellenanämie auf.

In einer weiter bevorzugten Ausgestaltung ist oder wurde die bereitgestellte Diskriminanzfunktion, insbesondere die bereitgestellten Signifikanzparameter, durch eine mehrfache Auswahl unterschiedlicher Dispersionen bekannter Klassifikation als jeweilige Berechnungsgrundlage und/oder durch eine mehrfache Auswahl der aus diesen Dispersionen ermittelten Trainingsmesswerten optimiert. Dieses Verfahren nimmt insbesondere darauf Bezug, dass gegebenenfalls nur ein relativ kleiner Datensatz von Trainingsmesswerten zur Verfügung steht, so dass die optimale Diskriminanzfunktion bzw. die optimalen Signifikanzparameter nicht mit einer ausreichend hohen statistischen Aussagekraft ermittelt werden können. Die Aussagekraft wird jedoch dadurch verbessert, indem aus dem zur Verfügung stehenden Datensatz von Trainingsmesswerten mehrfach beliebige Unterdatensätze von Trainingsmesswerten herausgegriffen und diese jeweils zur Berechnung einer Diskriminanzfunktion herangezogen werden. Mit anderen Worten werden die insgesamt zur Verfügung stehenden Trainingsmesswerte bzw. Dispersionen bekannter Klassifikation nicht als ein einzelner Datensatz zur Bestimmung der Diskriminanzfunktion eingesetzt, sondern es werden jeweils hieraus Unterdatensätze statistischer Zusammensetzung gebildet, die ihrerseits jeweils zur Berechnung einer optimierten Diskriminanzfunktion eingesetzt werden. Aus diesen mehrfachen Diskriminanzfunktionen kann dann beispielsweise mittels Testreihen die hinsichtlich der Klassifikation optimalste Diskriminanzfunktion ausgewählt werden.

Bevorzugt wird zur Bereitstellung der Diskriminanzfunktion daher aus den Dispersionen bekannter Klassifikation und/oder den hieraus ermittelten Trainingsmesswerten statistisch eine Mehrzahl von Untergruppen von Dispersionen und/oder von hieraus ermittelten Trainingsmesswerten herausgegriffen, wobei auf Basis der jeweiligen Untergruppen jeweils eine Diskriminanzfunktion, insbesondere die Auswahl der Signifikanzparameter ermittelt wird, und wobei aus den mehrfach ermittelten Diskriminanzfunktionen, insbesondere aus den mehrfach ermittelten Signifikanzparametern, diejenigen Diskriminanzfunktionen, insbesondere diejenigen Signifikanzparameter, bereitgestellt werden, die bezüglich der Klassifikation von Dispersionen bekannter Klassifikation eine Trefferquote oberhalb eines vorgegebenen Schwellwertes aufweisen. Dabei werden also die mehrfach ermittelten Diskriminanzfunktionen bzw. Signifikanzparameter einem Test unterzogen, indem diese hinsichtlich ihrer Vorhersagewahrscheinlichkeit auf Dispersionen bekannter Klassifikation angewendet werden. Auch für diese Testreihe können aus den bekannten Dispersionen wiederum jeweils Untergruppen ausgewählt werden.

Der Schwellwert kann beispielsweise über die Trefferquote als solche festgelegt werden, die die Anzahl der richtigen Klassifikationen (also sowohl hinsichtlich des Vorliegens einer Sichelzellenanämie als auch hinsichtlich des Vorliegens keiner Sichelzellenanämie) in Bezug auf die Anzahl der untersuchten Dispersionen bekannter Klassifikation gegeben ist. Alternativ kann der Schwellwert aber auch aus der Sensitivität oder Spezifität der jeweils ermittelten Diskriminanzfunktionen bestimmt werden. Dabei ist die Sensitivität als Wahrscheinlichkeit einer hinsichtlich der Sichelzellenanämie positiven Klassifikation definiert, gesetzt den Fall, dass diese Klassifikation korrekt ist. Die Spezifität ist definiert als Wahrscheinlichkeit einer hinsichtlich der Sichelzellenanämie negativen Klassifikation, gesetzt den Fall, dass diese Klassifikation richtig ist. Idealerweise sollte die gesuchte optimale Diskriminanzfunktion sowohl eine sehr hohe Spezifität als auch eine sehr hohe Sensitivität aufweisen.

In einer weiter bevorzugten Variante des Analyseverfahrens werden zur Optimierung der Diskriminanzfunktion, insbesondere zur Optimierung der Signifikanzparameter die Anzahl von optischen Merkmalen reduziert, insbesondere diejenigen optischen Merkmale entfernt, deren mehrfach ermittelte Signifikanzparameter einen vorgegebenen Schwellwert unterschreiten. In Trainings- bzw. Testreihen der vorbeschriebenen Art können beispielsweise in den jeweils ermittelten Diskriminanzfunktionen diejenigen optischen Merkmale ermittelt werden, die aufgrund sehr niedriger Signifikanzparameter keinen Beitrag zur Klassifikation liefern. Beim nächsten Testlauf werden diese optischen Merkmale dann aus der Diskriminanzfunktion entfernt und die Qualität der Klassifikation erneut beurteilt. Insgesamt kann dann auf diese Weise die Anzahl der für die Diskriminanzanalyse auszuwertenden optischen Merkmale auf ein sinnvolles Maß beschränkt werden, wobei die ausgeschlossenen optischen Merkmale keinen Einfluss auf die Qualität der Klassifikation haben.

Bevorzugt wird bei der Ermittlung der Messwerte im Rahmen des optischen Analyseverfahrens, insbesondere auch zur Ermittlung der Trainingsmesswerte, der Testdispersion, insbesondere auch der Dispersionen bekannter Klassifikation, ein Sphärungszusatz zur Rundung der Erythrozyten und/oder ein Fixermittel zur Haltbarmachung der Probe zugesetzt. Ein derartiger Sphärungszusatz ist beispielsweise Natriumlaurylsulfat (SDS = Sodiumdodecylsulfat). Ein Fixermittel ist beispielsweise Glutaraldehyd. Erstaunlicherweise hat sich herausgestellt, dass trotz Zusatz eines Sphärungsmittels die abnormale Sichelzellengestalt der betroffenen Erythrozyten nicht oder nur kaum merklich beeinflusst wird. Gleiches gilt für das Fixermittel. Insofern lässt sich das angegebene Verfahren problemlos für automatische optische Analyseverfahren, insbesondere in bekannten hämatologischen Analyseverfahren einsetzen. Dort werden Sphärungsmittel für Erythrozyten zugesetzt, um die Richtungsabhängigkeit der Streuung für Erythrozyten zu eliminieren. Bekanntermaßen haben gesunde Erytrozyten nicht die Gestalt einer Kugel sondern liegen in etwa mit einer Donut-Gestalt vor.

Die eingangs gestellte Aufgabe wird erfindungsgemäß auch durch ein Computerprogrammprodukt gelöst, welches einen Computer in die Lage versetzt, das vorbeschriebene Analyseverfahren auszuführen.

Weiter wird die gestellte Aufgabe erfindungsgemäß auch durch ein optisches Analysesystem, insbesondere durch ein hämatologisches Analysesystem, gelöst, welches eine Analyseeinrichtung zur Durchführung des vorbeschriebenen Analyseverfahrens umfasst.

Bevorzugt weist das optische Analysesystem eine Speichereinheit auf, in der die Diskriminanzfunktion, insbesondere die Signifikanzparameter für die jeweiligen optischen Merkmale und die statistischen Größen für die jeweiligen optischen Merkmale hinterlegt sind. Bevorzugt ist die Analyseeinrichtung eingerichtet, den zu untersuchenden Dispersionen ein Sphärungsmittel für Erythrozyten, insbesondere Natriumlaurylsulfat (SDS), und/oder ein Fixermittel, insbesondere Glutaraldehyd, zuzugeben.

Im Folgenden werden Ausführungsbeispiele der Erfindung anhand einer Zeichnung näher beschrieben und erläutert. Es zeigen:

FIG 1    Ein optisches Analysesystem gemäß dem Stand der Technik,

FIG 2    eine schematische Darstellung eines Teils des Datenflusses für ein optisches Analyseverfahren unter Verwendung einer Diskriminanzanalyse,

FIG 3    eine Darstellung von Signifikanzparametern zu einem jeweiligen optischen Merkmal, geordnet nach deren Betragsgröße,

FIG 4    ein Schema zur Ermittlung der optimalen Diskriminanzfunktion aus Trainingsmesswerten,

FIG 5-7     Auswertungen einer Vielzahl von durch eine Mehrfachauswahl aus Trainingsmesswerten ermittelten Diskriminanzfunktionen hinsichtlich der Sensitivität, der Spezifität und der Trefferquote, bezogen auf Dispersionen bekannter Klassifikation für das Vorliegen einer homozygoten oder heterozygoten Ausprägung der Sichelzellenanämie und

FIG 8-10    Auswertungen zur Auswahl der jeweils optimalsten Diskriminanzfunktion zur Klassifikation für das Vorliegen einer homozygoten oder heterozygoten Ausprägung der Sichelzellenanämie.

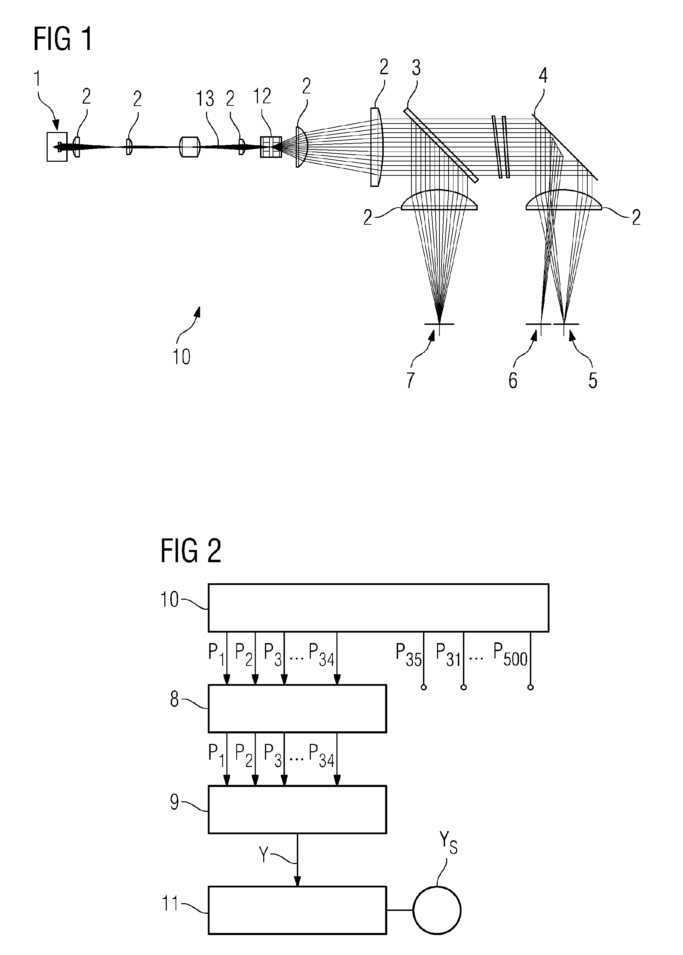

FIG 1 zeigt schematisch ein automatisch arbeitendes optisches Analysesystem 10 für biologische Proben, insbesondere für Blutproben. Das Analysesystem 10 umfasst eine Lichtquelle 1, wie zum Beispiel eine Laser-Lichtquelle, die einen Analysestrahl 13 generiert, der über verschiedene optische Elemente, insbesondere mittels Linsen 2, in eine Probe einer biologischen Dispersion 12 fokussiert wird. Nach Durchgang durch die zu untersuchende Dispersion 12 wird der aufgeweitete, abgelenkte oder gestreute Analysestrahl 13 durch weitere Linsen 2 gebündelt und mittels eines Strahlteilers 3 in einen spektralen Sensor 7, wie zum Beispiel einem Spektrometer, geführt. Über einen Spiegel 4 werden Fernfeldbeugungsmuster 5 und Nahfeldbeugungsmuster 6 in jeweils einem Sensor detektiert. Auf diese Weise werden eine Vielzahl von Messwerten, die einem jeweiligen optischen Merkmal der untersuchten Dispersion 12 zuzuordnen sind, auf automatisierte Art und Weise bestimmt.

FIG 2 zeigt für ein optisches Analysesystem 10 einen Teil des Datenflusses zur Verwendung einer Diskriminanzanalyse zur Klassifikation der untersuchten Dispersion hinsichtlich des Vorliegens einer spezifischen Erkrankung, insbesondere der Sichelzellenanämie. Das Analysesystem 10 ermittelt zunächst automatisiert aus einer Blutprobe, insbesondere unter Zugabe von Hilfsmitteln wie Färbungsmitteln, Verdünnungsmitteln, oder Sphärungsmitteln, eine Anzahl n von Messwerten $P_i$ zu jeweils konkret bestimmten optischen Merkmalen. Vorliegend werden automatisiert 500 optische Merkmale der untersuchten Dispersion analysiert bzw. deren Messwerte ermittelt. Zur Klassifikation hinsichtlich des Vorliegens einer Sichelzellenanämie werden die ausgewählten Messwerte $P_1$ bis $P_{34}$ aus dem optischen Analyseverfahren in einer nachgeschalteten Datenverarbeitung 8, die beispielsweise in einem Computer vorgenommen werden kann, gegebenenfalls aufbereitet und zur Berechnung eines Klassfikationsindex Y herangezogen, wozu eine konkrete Diskriminanzfunktion zur Klassifikation der Sichelzellenanämie hinterlegt ist. Der bestimmte Klassifikationsindex Y wird mit einem ebenfalls hinterlegten Schwellwert $Y_S$ verglichen. Abschließend wird dem Benutzer angezeigt, ob der Schwellwert über- oder unterschritten ist. Demnach wird automatisiert angezeigt, ob die untersuchte Dispersion hinsichtlich des Vorliegens einer Sichelzellenanämie positiv oder negativ klassifiziert ist.

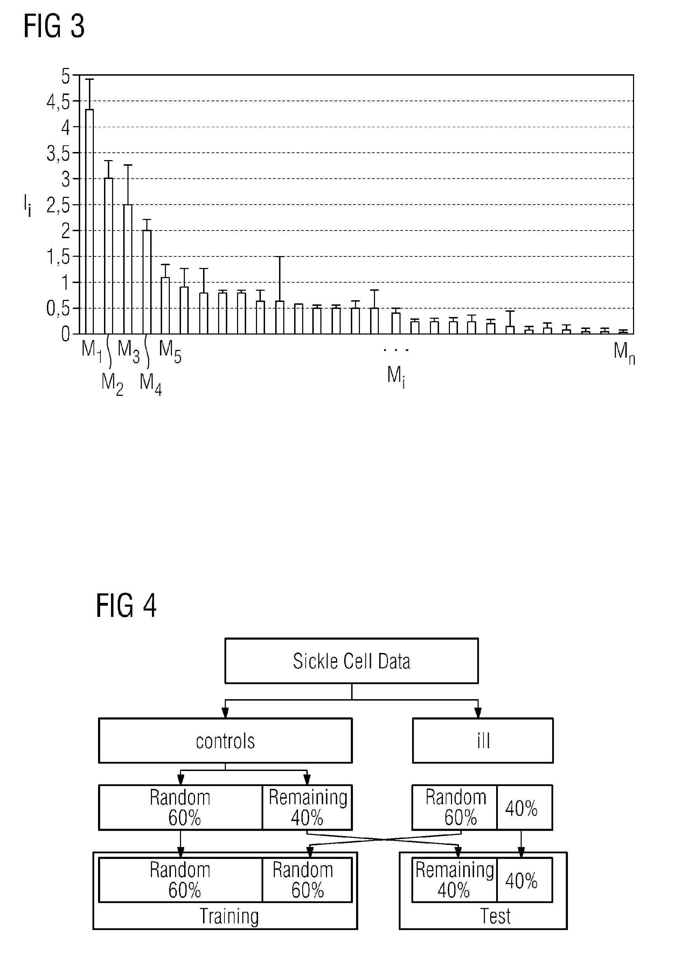

FIG 3 zeigt ein Diagramm mit für eine hinterlegte oder bereitgestellte Diskriminanzfunktion anhand von Trainingsläufen ermittelten Signifikanzparametern $I_i$, wobei die Signifikanzparameter $I_i$ über das jeweils zu analysierende bzw. zu messende optische Merkmal $M_i$ aufgetragen sind. Dabei sind die optischen Merkmale $M_i$ bezüglich des Wertes des zugehörigen Signifikanzparameters $I_i$ absteigend sortiert. Das Diagramm der FIG 3 zeigt beispielsweise die optischen Merkmale $M_i$ zu den Messwerten $P_i$, die in FIG 2 gezeigt sind. Man erkennt, dass viele optische Merkmale $M_i$ eine vergleichsweise niedrige und insofern hinsichtlich der Diskriminanzanalyse bezüglich des Vorliegens einer Sichelzellenanämie vernachlässigbare Gewichtung bzw. Signifikanz aufweisen.

FIG 4 zeigt ein Schema zur Ermittlung der optimalen Diskriminanzfunktion hinsichtlich des Vorliegens einer Sichelzellenanämie aus Trainingsmesswerten. Zunächst liegt ein Datensatz (Sickle Cell Data) aus Trainingsmesswerten zu Dispersionen bekannter Klassifikation vor. Demnach ist bekannt, welche Messwerte zu Proben oder Dispersionen von an Sichelzellenanämie erkrankten Personen gehören, und welche Messwerte Proben oder Dispersionen gesunder Personen zugeordnet sind. Entsprechend werden aus dem Datensatz zwei Gruppen von Messwerten, nämlich eine Kontrollgruppe (Controls) und eine Erkrankungsgruppe (Ill) gebildet bzw. liegen die Messwerte bereits entsprechend sortiert vor. Aus den beiden Gruppen werden jeweils statistisch Untergruppen von Datensätzen bzw. Messwerten herausgegriffen und zu einer Trainingsgruppe (Training) und zu einer Testgruppe (Test) miteinander vermischt. Im gezeigten Ausführungsbeispiel werden hierzu statistisch aus den Messreihen der Kontrollgruppe 60 % der Datensätze und aus den Messreihen der Erkrankungsgruppe 40 % der Datensätze gewählt. Die jeweils 60 % ausgewählten Datensätze werden zur Trainingsgruppe gemischt. Die verbleibenden jeweils 40 % der Datensätze bilden die Testgruppe.

Zur Ermittlung der optimalen Diskriminanzfunktion wird der in FIG 4 gezeigte Prozess vielfach (z.B. 1000-fach) wiederholt und für die jeweils gebildete Trainingsgruppe jeweils eine spezifisch optimierte Diskriminanzfunktion bzw. die hierzu mathematisch aus der zugrundeliegenden Optimierungsaufgabe resultierenden Signifikanzparameter ermittelt. Mit anderen Worten wird für jeden durch die jeweilige Trainingsgruppe gebildeten Datensatz mittels einer Diskriminanzanalyse die hinsichtlich einer Klassifikation in die Kontrollgruppe und die Erkrankungsgruppe optimale Diskriminanzfunktion ermittelt. Dabei werden insbesondere aus den zugrundeliegenden Trainingsmesswerten die für die beiden Gruppen jeweils relevanten statistischen Größen wie Mittelwerte, Erwartungswerte, Varianzen, Standardabweichungen oder auch Kovarianzen bestimmt. Jede derart ermittelte Diskriminanzfunktion wird abgelegt und ihre Qualität hinsichtlich der Klassifikation für das Vorliegen der Sichelzellenanämie anhand der jeweiligen Datensätze der Testgruppe analysiert. Dazu wird die jeweilige Diskriminanzfunktion auf die einer jeweiligen Dispersion jeweils konkret zugeordneten Messwerte aus dem Datensatz der Testgruppe angewendet und die über den jeweils berechneten Klassifikationsindex vorhergesagte Klassifikation überprüft. Insgesamt kann so eine Aussage über die Trefferquote der jeweils ermittelten Diskriminanzfunktion sowie über deren Sensitivität und Spezifität gemacht werden.

Entsprechend dem in FIG 4 gezeigten Ablaufschema wird auch eine Reduzierung der zur Klassifikation herangezogenen optischen Merkmale vorgenommen, die durch das jeweilige Analyseverfahren als Ausgangsparameter zur Verfügung gestellt werden. Für jede der zahlreichen ermittelten Diskriminanzfunktionen können nämlich die jeweils optischen Merkmale entsprechend FIG 3 bezüglich ihrer Signifikanzparameter bewertet bzw. sortiert werden. Die mit der jeweils geringsten Signifikanz versehenen optischen Merkmale wurden aus der Diskriminanzfunktion entfernt und ihre Qualität erneut bewertet. Sofern sich die Qualität der Klassifikation nicht verändert, können die optischen Merkmale zur Klassifikation hinsichtlich des Vorliegens der Sichelzellenanämie entfernt werden. Auf diese Weise konnte die Anzahl der zur Klassifikation erforderlichen optischen Merkmale von 500, die beispielsweise das hämatologische automatische Analysesystem ADVIA zur Verfügung stellt, auf etwa 30 reduziert werden.

Die zur Klassifikation bezüglich des Vorliegens einer Sichelzellenanämie noch verbliebenen optischen Merkmale sind aus der folgenden Tabelle ersichtlich:

| I | ADVIA Parameter/ optisches Merkmal $M_i$ | Definition des Merkmals $M_i$ zum jeweiligen Merkmal i |
|---|---|---|
| 1 | WBCB | Zählung der weißen Blutkörperchen aus dem Baso-Kanal |
| 2 | RBC | Zählung der roten Blutkörperchen aus dem RBC/PLT-Kanal |
| 3 | measHGB | Gemessene Hämoglobin-Konzentration |
| 4 | MCV | Mittleres Zellvolumen der gezählten roten Blutkörperchen (RBC) |
| 5 | CHCM | Mittlere Hämoglobinkonzentration (g/dL) der Zellen (aus RBC) |
| 6 | RDW | Dichteverteilungsbreite der roten Blutkörperchen (aus RBC) |
| 7 | HDW | Breite der Hämoglobindichteverteilung (g/dL) |
| 8 | MN_y_peak | Peak Y Kanal des Monocyten-Clusters aus dem Baso-Kanal |
| 9 | plat mode | PLT Mode |
| 10 | mu_fit | $\mu$-FIT |
| 11 | sig_fit | $\sigma$-FIT |
| 12 | micro_pcnt | Anzahl der RBC Zellen in Prozent mit weniger als 60 Femtoliter |
| 13 | macro_pcnt | Anzahl der RBC Zellen in Prozent mit mehr als 120 Femtoliter |
| 14 | hypo_pcnt | Anzahl der von roten Blutkörperchen in Prozent mit einer Hämoglobin Konzentration von weniger als 28 g/dL |
| 15 | hyper_pcnt | Anzahl der von roten Blutkörperchen in Prozent mit einer Hämoglobin Konzentration von weniger als 41 g/dL |
| 16 | H_mean | Mittelwert der Hämoglobinkonzentration in Pikogramm |
| 17 | H_deviation | Breite der Hämoglobininhaltsverteilung in Pikogramm |
| 18 | VHC_covar | Kovarianz der Hämoglobindichteverteilung |
| 19 | pcnt_blasts | Anteil der weißen Blutkörperchen gezählt im Blast-Cluster aus dem Baso-Kanal |
| 20 | pcnt_mns | Anteil der weißen Blutkörperchen gezählt im Monozyten-Cluster aus dem Baso-Kanal |
| 21 | pcnt_pmns | Anteil der weißen Blutkörperchen gezählt im Cluster der Polymorphkernigen aus dem Baso-Kanal |
| 22 | pcnt_baso_noise | Anteil der Ereignisse im Baso-Rausch-Cluster relativ zur Gesamtzahl an Signalereignissen im Baso-Kanal |
| 23 | PLT | Zählung der erkannten Blutplättchen |
| 24 | MPV | Mittleres Blutplättchenvolumen |

(fortgesetzt)

| I | ADVIA Parameter/ optisches Merkmal $M_i$ | Definition des Merkmals $M_i$ zum jeweiligen Merkmal i |
|---|---|---|
| 25 | PDW | Blutplättchenverteilungsbreite |
| 26 | MPC | Mittlere Blutplättchen- Komponentenkonzentration |
| 27 | MPM | Mittlere Blutplättchentrockenmasse |
| 28 | PMDW | Verteilungsbreite der Blutplättchentrockenmasse |
| 29 | PLT_Mean_X | Mittelwert der Beugungsablenkung in X-Richtung |
| 30 | PLT_Mean_Y | Mittelwert der Beugungsablenkung in Y-Richtung |
| 31 | Large_PLT | große Blutplättchen (Anzahl) |
| 32 | RBC_Fragments | Anzahl der gezählten Fragmente im PLT-Beugungszytogramm |
| 33 | RBC_Ghosts | Anzahl der Ereignisse im PLT-Beugungszytogramm ohne Zuordnung |
| 34 | n_fit | Kurveneinpassung im 1D Blutplättchen-Histogramm |

Dabei sind die ersten optischen Merkmale 1 bis 33 relevant für die Klassifikation hinsichtlich des Vorliegens einer homozygoten Sichelzellenanämie. Das optische Merkmal Nr. 34 wird hinzugenommen bei einer Klassifikation hinsichtlich des Vorliegens einer heterozygoten Sichelzellenanämie. Hinsichtlich ihrer Signifikanz sind die Merkmale mit der Nr. 1 bis 11 sehr relevant. Die folgenden Merkmale bis zum Merkmal mit der Nr. 24 sind relevant und tragen eindeutig zur Verbesserung der Qualität der Diskriminanzfunktion bei. Die Merkmale mit der Nr. 25 bis 34 weisen nur eine geringe Signifikanz auf. Jedoch tragen auch sie noch zu einer Verbesserung der Qualität der Diskriminanzfunktion bei.

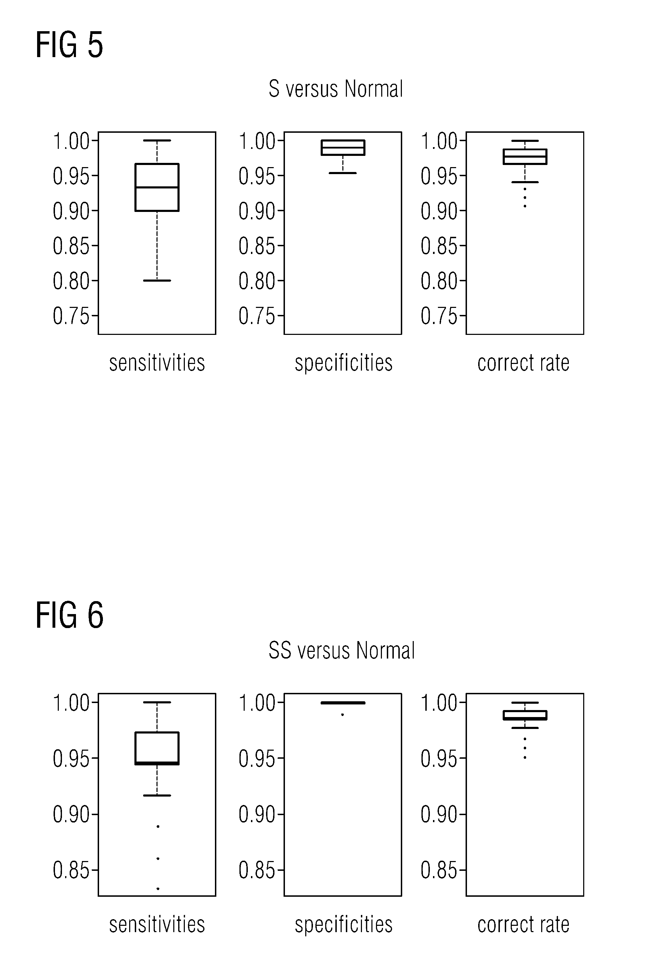

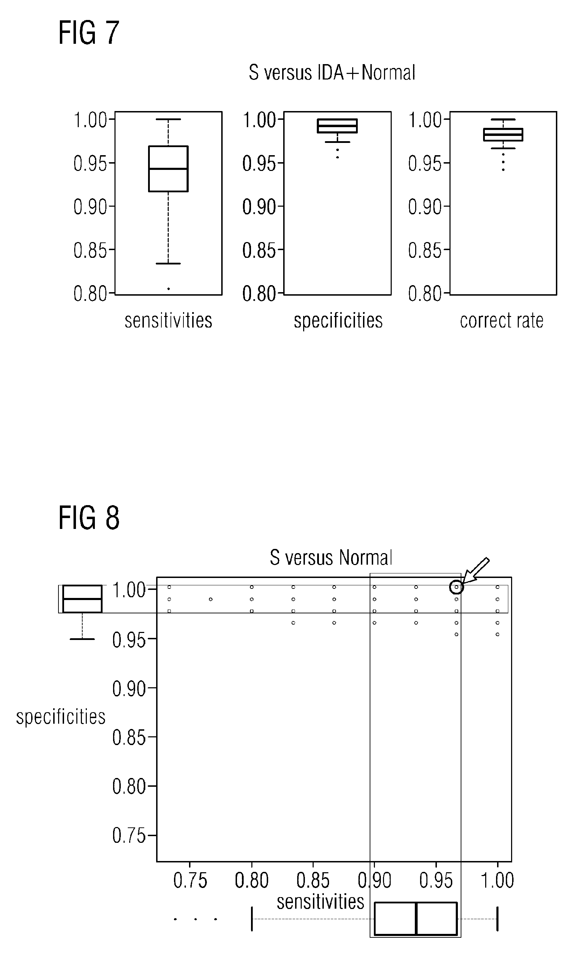

In den FIG 5 bis 7 sind Auswertungen der jeweils gemäß dem Verlaufsschema in FIG 4 ermittelten Diskriminanzfunktionen hinsichtlich ihrer Sensitivität, Spezifität und Trefferquote dargestellt. In FIG 5 wurden Datensätze aus Dispersionen bekannter Diagnose einer heterozygoten Sichelzellenanämie (S) und Dispersionen gesunder Personen ausgewertet. FIG 6 bezieht sich auf Datensätze für Dispersionen bekannter Diagnose einer homozygoten Sichelzellenanämie (SS) und auf Dispersionen gesunder Personen. FIG 7 bezieht sich auf eine entsprechende Analyse der Diskriminanzfunktionen aus Datensätzen zu Dispersionen bekannter Klassifikation hinsichtlich des Vorliegens einer homozygoten Sichelzellenanämie (SS) und Dispersionen von gesunden Personen, die zusätzlich einen Eisenmangel (IDA) aufwiesen.

In allen FIG 5 bis 7 sind die Auswertungen in Form sogenannter Box-Plots dargestellt. Dabei ist jeweils als durchgezogene Linie innerhalb der Box der Median eingezeichnet. Die Box selbst beinhaltet 25 % der Messwerte unterhalb und 25 % der Messwerte oberhalb des Medians (der sogenannte Inter-Quartillen-Bereich). Die jeweiligen Endmarkierungen beziehen sich auf die Spreizung der Messwerte. Ausreißer sind teils eingezeichnet, jedoch für die Darstellung des Box-Plots nicht berücksichtigt. Es wird ersichtlich, dass die jeweils entsprechend dem Schema nach FIG 4 durch statistische Untergruppenbildung ermittelten Diskriminanzfunktionen allesamt hinsichtlich des Vorliegens einer Sichelzellenanämie hohe Qualität zeigen. Die Sensitivitäten innerhalb der jeweiligen Box, also im Inter-Quartillen-Bereich bewegen sich alle oberhalb von 0,9. Die Spezifitäten und Trefferquoten im Inter-Quartillen-Bereich liegen alle oberhalb von 0,95.

Es empfiehlt sich, für die jeweils dem Analyseverfahren bereitgestellten optimalsten Diskriminanzfunktionen diejenigen auszuwählen, die eine Sensitivität und Spezifität im Inter-Quartillen-Bereich aufweisen. Die optimalste Diskriminanzfunktion ergibt sich als diejenige Diskriminanzfunktion, die die jeweils größte Spezifität und jeweils größte Sensitivität aufweist.

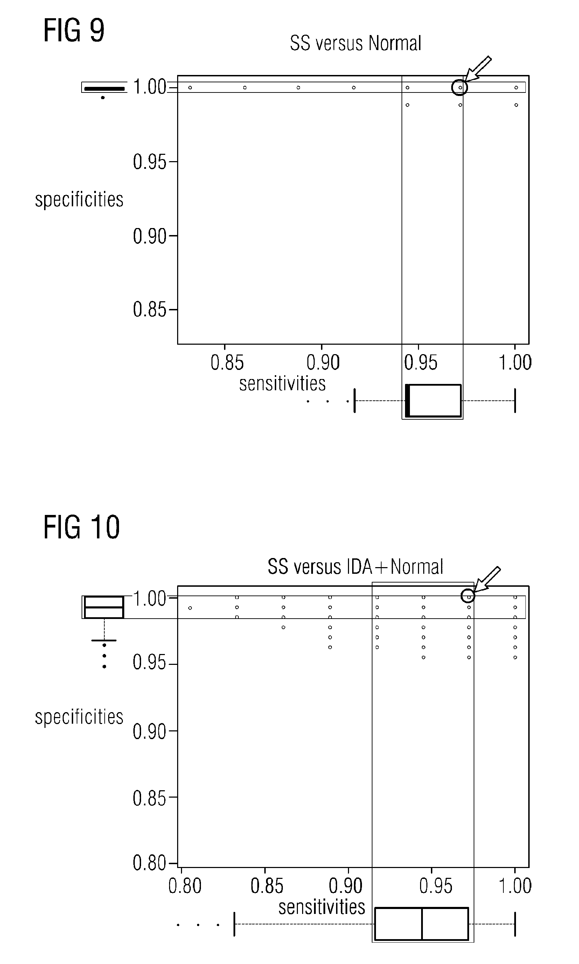

In den FIG 8 bis 10 ist hierzu eine Auswertung der untersuchten Diskriminanzfunktionen entsprechend den FIG 5 bis 7 dargestellt. Dabei sind jeweils die Box-Plots der Sensitivitäten und Spezifitäten gegeneinander aufgetragen. Die dem optischen Analyseverfahren zur Klassifikation hinsichtlich des Vorliegens einer Sichelzellenanämie zugrunde zu legenden Diskriminanzfunktionen sind diejenigen Diskriminanzfunktionen, die im Überschneidungsbereich der Inter-Quartillen-Bereiche von Sensitivität und Spezifität liegen. Die optimalste Diskriminanzfunktion ist als diejenige Diskriminanzfunktion gekennzeichnet, die im Inter-Quartillen-Bereich die größte Sensitivität und die größte Spezifität aller untersuchten Diskriminanzfunktionen aufweist.

**Patentansprüche**

1. Analyseverfahren zur Klassifikation einer Erythrozyten enthaltenden Testdispersion hinsichtlich des Vorliegens einer Sichelzellenanämie mit den Schritten

   - Ermittlung von Messwerten ($P_i$) zu optischen Merkmalen ($M_i$) der zu klassifizierenden, Erythrozyten enthaltenden Testdispersion (12),
   - Berechnung eines Klassifikationsindex (Y) mittels einer bereitgestellten, insbesondere linearen, Diskriminanzfunktion unter Verwendung einer Anzahl (n) der ermittelten Messwerte ($P_i$) und
   - Klassifikation der Testdispersion (12) hinsichtlich des Vorliegens der Sichelzellenanämie in Abhängigkeit vom berechneten Klassifikationsindex (Y).

2. Analyseverfahren nach Anspruch 1, wobei der Klassifikationsindex (Y) aus einer Anzahl (n) der Messwerte ($P_i$) zu den jeweiligen optischen Merkmalen ($M_i$) mittels durch die Diskriminanzfunktion für die optischen Merkmale ($M_i$) bereitgestellten Signifikanzparametern ($I_i$) ermittelt wird.

3. Analyseverfahren nach Anspruch 1 oder 2, wobei der Klassifikationsindex (Y) mittels durch die Diskriminanzfunktion für die optischen Merkmale ($M_i$) bereitgestellten statistischen Größen, insbesondere Mittelwerte ($E_i$) und Standardabweichungen ($\sigma_i$), ermittelt wird.

4. Analyseverfahren nach Anspruch 2 und 3, wobei die Signifikanzparameter ($I_i$) und die statistischen Größen aus Trainingsmesswerten zu den optischen Merkmalen ($M_i$) in Dispersionen bekannter Klassifikation ermittelt wurden.

5. Analyseverfahren nach Anspruch 4, wobei die statistischen Größen der optischen Merkmale ($M_i$) aus ersten Trainingsmesswerten ermittelt wurden, wobei die ersten Trainingsmesswerte von Dispersionen mit einer bezüglich des Vorliegens der Sichelzellenanämie negativen Klassifikation und die zweiten Trainingsmesswerte von Dispersionen mit einer bezüglich des Vorliegens der Sichelzellenanämie positiven Klassifikation abgeleitet wurden.

6. Analyseverfahren nach Anspruch 5, wobei die Signifikanzparameter ($I_i$) aus den ersten und aus den zweiten Trainingsmesswerten mittels einer Diskriminanzanalyse abgeleitet sind, wobei bei der Diskriminanzanalyse insbesondere ein Bayes'sches Theorem verwendet wird.

7. Analyseverfahren nach einem der Ansprüche 1 bis 6, wobei ein Schwellwert ($Y_S$) zur Klassifikation verwendet wird, wobei hinsichtlich des Vorliegens der Sichelzellenanämie ein Wert des Klassifikationsindex von $Y > Y_S$ einer positiven Klassifikation und von $Y < Y_S$ einer negativen Klassifikation zugeordnet ist.

8. Analyseverfahren nach einem der Ansprüche 1 bis 7, wobei zur Berechnung des Klassifikationsindex (Y) wenigstens zwei optische Merkmale berücksichtigt werden, und wobei ein erstes optisches Merkmal ein Maß für eine optische Streuung in eine transversale X-Richtung zu einem Analysestrahl (13) ist und ein zweites optisches Merkmal ein Maß für eine optische Streuung in einer zur X-Richtung senkrechten und zum Analysestrahl (13) senkrechten Y-Richtung ist, wobei der Analysestrahl (13) durch die Testdispersion (12) verläuft.

9. Analyseverfahren nach einem der Ansprüche 1 bis 8, wobei die bereitgestellte Diskriminanzfunktion, insbesondere die bereitgestellten Signifikanzparameter ($I_i$), durch eine mehrfache Auswahl unterschiedlicher Dispersionen bekannter Klassifikation als jeweilige Berechnungsgrundlage und/oder durch eine mehrfache Auswahl der aus diesen Dispersionen ermittelten Trainingsmesswerte optimiert wurde.

10. Analyseverfahren nach Anspruch 9, wobei aus den Dispersionen bekannter Klassifikation und/oder den hieraus ermittelten Trainingsmesswerten statistisch eine Mehrzahl von Untergruppen von Dispersionen und/oder von hieraus ermittelten Trainingsmesswerten herausgegriffen wurde, wobei auf Basis der jeweiligen Untergruppen jeweils eine Diskriminanzfunktion, insbesondere die Auswahl der Signifikanzparameter ($I_i$), ermittelt wurde, und wobei aus den mehrfach ermittelten Diskriminanzfunktionen, insbesondere aus den mehrfach ermittelten Signifikanzparametern ($I_i$), diejenigen Diskriminanzfunktionen, insbesondere diejenigen Signifikanzparameter ($I_i$), bereitgestellt wurden, die bezüglich der Klassifikation von Dispersionen bekannter Klassifikation eine Trefferquote oberhalb eines vorgegebenen Schwellwertes aufweisen.

11. Analyseverfahren nach Anspruch 10, wobei die Trefferquote der mehrfach ermittelten Diskriminanzfunktionen an statistisch aus den Dispersionen bekannter Klassifikation herausgegriffenen Untergruppen von Dispersionen ermittelt wurde.

**12.** Analyseverfahren nach einem der Ansprüche 9 bis 11, wobei zur Optimierung der Diskriminanzfunktion, insbesondere zur Optimierung der Signifikanzparameter ($I_i$) die Anzahl (n) von optischen Merkmalen ($M_i$) reduziert wurde, insbesondere diejenigen optischen Merkmale ($M_i$) entfernt wurden, deren mehrfach ermittelte Signifikanzparameter ($I_i$) einen vorgegebenen Schwellwert unterschreiten.

**13.** Analyseverfahren nach einem der Ansprüche 1 bis 12, wobei zur Ermittlung der Messwerte ($P_i$), insbesondere auch zur Ermittlung der Trainingsmesswerte, der Testdispersion (12), insbesondere auch der Dispersionen bekannter Klassifikation, ein Sphärungsmittel für Erythrozyten, insbesondere Natriumlaurylsulfat (SDS), und/oder ein Fixierungsmittel, insbesondere Glutaraldehyd, zugesetzt wird.

**14.** Computerprogrammprodukt, welches einen Computer in die Lage versetzt, das Analyseverfahren nach einem der Ansprüche 1 bis 13 auszuführen.

**15.** Optisches Analysesystem (10), insbesondere ein hämatologisches Analysesystem (10), mit einer Analyseeinrichtung zur Durchführung des Analyseverfahrens nach einem der Ansprüche 1 bis 13.

**16.** Optisches Analysesystem (10) nach Anspruch 15, mit einer Speichereinheit, in der die Diskriminanzfunktion, insbesondere die Signifikanzparameter ($I_i$) und die statistischen Größen für die jeweiligen optischen Merkmale ($M_i$), insbesondere die Mittelwerte ($E_i$) und die Standardabweichungen ($\sigma_i$), hinterlegt sind.

**17.** Optisches Analysesystem (10) nach Anspruch 15 oder 16, wobei die Analyseeinrichtung eingerichtet ist, den zu untersuchenden Dispersionen ein Sphärungsmittel für Erythrozyten, insbesondere Natriumlaurylsulfat (SDS), und/oder ein Fixiermittel, insbesondere Glutaraldehyd, zuzugeben.

FIG 1

FIG 2

FIG 3

FIG 4

## FIG 5

### S versus Normal

sensitivities     specificities     correct rate

## FIG 6

### SS versus Normal

sensitivities     specificities     correct rate

## FIG 7

S versus IDA + Normal

sensitivities    specificities    correct rate

## FIG 8

S versus Normal

specificities

sensitivities

## FIG 9

SS versus Normal

specificities

sensitivities

## FIG 10

SS versus IDA + Normal

specificities

sensitivities

Europäisches Patentamt

European Patent Office

Office européen des brevets

Nummer der Anmeldung

EP 15 16 2478

**EUROPÄISCHER RECHERCHENBERICHT**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | DE 10 2013 216362 A1 (SIEMENS HEALTHCARE DIAGNOSTICS [DE]) 19. Februar 2015 (2015-02-19) | 1-12, 14-16 | INV. G06K9/00 G01N33/49 G01N21/49 G06K9/62 |
| Y | * das ganze Dokument * ----- | 13,17 | |
| Y | "ADVIA 2120/2120i Hematology Systems Operator's Guide", , 2010, XP055211300, Gefunden im Internet: URL:http://photos.medwrench.com/equipmentManuals/2029-4080.pdf [gefunden am 2015-09-04] * Seite 1.9 * * Seite 8.37 * * Seite 9.29 * ----- | 13,17 | ADD. G01N15/14 |
| X | GUAL-ARNAU X ET AL: "Erythrocyte shape classification using integral-geometry-based methods", MEDICAL AND BIOLOGICAL ENGINEERING AND COMPUTING, SPRINGER, HEILDELBERG, DE, Bd. 53, Nr. 7, 13. März 2015 (2015-03-13), Seiten 623-633, XP035503679, ISSN: 0140-0118, DOI: 10.1007/S11517-015-1267-X [gefunden am 2015-03-13] * das ganze Dokument * ----- -/-- | 1,2, 9-11, 14-16 | RECHERCHIERTE SACHGEBIETE (IPC) G06K G01N |

Der vorliegende Recherchenbericht wurde für alle Patentansprüche erstellt

| Recherchenort | Abschlußdatum der Recherche | Prüfer |
|---|---|---|
| Den Haag | 7. September 2015 | Hermes, Lothar |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

.......................................................................................
& : Mitglied der gleichen Patentfamilie, übereinstimmendes Dokument

EPO FORM 1503 03.82 (P04C03)

Europäisches
Patentamt

European
Patent Office

Office européen
des brevets

# EUROPÄISCHER RECHERCHENBERICHT

**Nummer der Anmeldung**

EP 15 16 2478

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | NITHYAA A N ET AL: "Chapter 34 -Automated detection and classification of blood diseases", RECENT ADVANCEMENTS IN SYSTEM MODELLING APPLICATIONS : PROCEEDINGS OF NATIONAL SYSTEMS CONFERENCE 2012 / EDITED BY R MALATHI, J KRISHNAN; [LECTURE NOTES IN ELECTRICAL ENGINEERING 1876-1100], Bd. 188, 2013, Seiten 393-404, XP008177380, DOI: 10.1007/978-81-322-1035-1_34 ISBN: 978-81-322-1034-4 [gefunden am 2013-03-08] * Zusammenfassung * * Abbildungen 34.6-34.10 * ----- | 1-5, 14-16 | |
| X | US 6 630 990 B2 (OEVER RONNY VAN T [NL] ET AL) 7. Oktober 2003 (2003-10-07) * Spalte 1, Zeilen 35-54 * * Spalte 11, Zeilen 1-17 * * Spalte 13, Zeilen 17-33 * ----- | 1-4,7, 14-16 | |
| A | YOUNG RAN KIM ET AL: "Automated red blood cell differential analysis on a multi-angle light scatter/fluorescence hematology analyzer", CYTOMETRY, Bd. 56B, Nr. 1, 21. Oktober 2003 (2003-10-21), Seiten 43-54, XP055211503, ISSN: 0196-4763, DOI: 10.1002/cyto.b.10048 * das ganze Dokument * * Seiten 45-51 * ----- -/-- | 1,13-15, 17 | |

**RECHERCHIERTE SACHGEBIETE (IPC)**

Der vorliegende Recherchenbericht wurde für alle Patentansprüche erstellt

| Recherchenort | Abschlußdatum der Recherche | Prüfer |
|---|---|---|
| Den Haag | 7. September 2015 | Hermes, Lothar |

EPO FORM 1503 03.82 (P04C03)

**Europäisches Patentamt**
**European Patent Office**
**Office européen des brevets**

# EUROPÄISCHER RECHERCHENBERICHT

Nummer der Anmeldung

EP 15 16 2478

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | GUYON I ET AL: "GENE SELECTION FOR CANCER CLASSIFICATION USING SUPPORT VECTOR MACHINES", MACHINE LEARNING, KLUWER ACADEMIC PUBLISHERS, BOSTON, US, Bd. 46, Nr. 1-03, 2002, Seiten 389-422, XP001205254, ISSN: 0885-6125, DOI: 10.1023/A:1012487302797 * Seite 393 * ----- | 12 | |
| | | | **RECHERCHIERTE SACHGEBIETE (IPC)** |

Der vorliegende Recherchenbericht wurde für alle Patentansprüche erstellt

| Recherchenort | Abschlußdatum der Recherche | Prüfer |
|---|---|---|
| Den Haag | 7. September 2015 | Hermes, Lothar |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

............................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 15 16 2478

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

07-09-2015

| Im Recherchenbericht angeführtes Patentdokument | Datum der Veröffentlichung | Mitglied(er) der Patentfamilie | Datum der Veröffentlichung |
|---|---|---|---|
| DE 102013216362 A1 | 19-02-2015 | DE 102013216362 A1<br>WO 2015024770 A1 | 19-02-2015<br>26-02-2015 |
| US 6630990 B2 | 07-10-2003 | CA 2449401 A1<br>EP 1399728 A2<br>JP 4184258 B2<br>JP 4814912 B2<br>JP 2004535570 A<br>JP 2008268230 A<br>US 2003025896 A1<br>WO 02099380 A2 | 12-12-2002<br>24-03-2004<br>19-11-2008<br>16-11-2011<br>25-11-2004<br>06-11-2008<br>06-02-2003<br>12-12-2002 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 0058727 A